# EUROPEAN PATENT SPECIFICATION

(11) **EP 4 331 595 B1**
(45) Date of publication and mention of the grant of the patent: **21.08.2024**
(21) Application number: 22919275.2
(22) Date of filing: 13.07.2022
(51) Int. Cl.: A61K 35/30, C12N 5/074, C12N 5/0735, A61P 27/02, A61K 35/545

(54) **USE OF RETINAL PIGMENT EPITHELIAL CELLS IN REPLACEMENT OF CORNEAL ENDOTHELIA**
VERWENDUNG VON RETINALEN PIGMENTEPITHELZELLEN BEIM ERSATZ DES HORNHAUTENDOTHELS
UTILISATION DE CELLULES ÉPITHÉLIALES PIGMENTAIRES RÉTINIENNES POUR LE REMPLACEMENT DE L'ENDOTHÉLIUM CORNÉEN

(43) Date of publication of application: 06.03.2024
(73) Proprietor: Eye Institute of Shandong First Medical University, Qingdao, Shandong 266071 (CN)
(72) Inventor: SHI, Weiyun, Qingdao, Shandong 266071 (CN); ZHOU, Qingjun, Qingdao, Shandong 266071 (CN); LI, Zongyi, Qingdao, Shandong 266071 (CN); DONG, Chunxiao, Qingdao, Shandong 266071 (CN); DUAN, Haoyun, Qingdao, Shandong 266071 (CN)
(74) Representative: Mewburn Ellis LLP
(86) International application number: PCT/CN2022/105465
(87) International publication number: WO 2024/011449

(56) References cited:
- CN-A- 105 838 676
- CN-A- 106 609 256
- CN-A- 108 728 413
- CN-A- 109 136 184
- CN-A- 110 628 696
- CN-A- 113 699 097
- US-A1- 2016 122 711
- LI ZONGYI ET AL: "Rapid Differentiation of Multi-Zone Ocular Cells from Human Induced Pluripotent Stem Cells and Generation of Corneal Epithelial and Endothelial Cells", STEM CELLS AND DEVELOPMENT, vol. 28, no. 7, 1 April 2019 (2019-04-01), US, pages 454 - 463, XP093113140, ISSN: 1547-3287, DOI: 10.1089/scd.2018.0176
- SURENDRAN HARSHINI ET AL: "Transplantation of retinal pigment epithelium and photoreceptors generated concomitantly via small molecule-mediated differentiation rescues visual function in rodent models of retinal degeneration", STEM CELL RESEARCH & THERAPY, vol. 12, no. 1, 1 January 2021 (2021-01-01), XP055835678, Retrieved from the Internet <URL:https://stemcellres.biomedcentral.com/track/pdf/10.1186/s13287-021-02134-x.pdf> DOI: 10.1186/s13287-021-02134-x
- DONG CHUNXIAO ET AL: "Ex vivo cultivated retinal pigment epithelial cell transplantation for the treatment of rabbit corneal endothelial dysfunction", EYE AND VISION, vol. 10, no. 1, 1 August 2023 (2023-08-01), London, UK, pages 1 - 16, XP093113381, ISSN: 2326-0254, Retrieved from the Internet <URL:https://www.ncbi.nlm.nih.gov/pmc/articles/PMC10394777/pdf/40662_2023_Article_351.pdf> DOI: 10.1186/s40662-023-00351-4
- GUO XIAOLING, ZHU DELIANG, LIAN RUILING, ZENG QIAOLANG, MATHEW SANJANA, TANG SHIBO, CHEN JIANSU: "Spheroid transplantable and functional retinal pigment epithelium derived from non-colony dissociated human induced pluripotent stem cells", RESEARCHSQAURE, 23 January 2020 (2020-01-23), XP093086167, Retrieved from the Internet <URL:https://assets.researchsquare.com/files/rs-12457/v2/c5f74267-db02-4297-ab85-f7c9c13c4fd7.pdf?c=1631841354> [retrieved on 20230926], DOI: 10.21203/rs.2.21874/v2
- ISHIDA MASAAKI, SUGITA SUNAO, MAKABE KENICHI, FUJII SHOTA, FUTATSUGI YOKO, KAMAO HIROYUKI, YAMASAKI SUGURU, SAKAI NORIKO, MAEDA AK: "A ROCK Inhibitor Promotes Graft Survival during Transplantation of iPS-Cell-Derived Retinal Cells", INTERNATIONAL JOURNAL OF MOLECULAR SCIENCES, vol. 22, no. 6, pages 3237, XP055898489, DOI: 10.3390/ijms22063237
- CROZE ROXANNE H., BUCHHOLZ DAVID E., RADEKE MONTE J., THI WILLIAM J., HU QIRUI, COFFEY PETER J., CLEGG DENNIS O.: "ROCK Inhibition Extends Passage of Pluripotent Stem Cell-Derived Retinal Pigmented Epithelium", STEM CELLS TRANSLATIONAL MEDICINE, ALPHAMED PRESS, INC., US, vol. 3, no. 9, 1 September 2014 (2014-09-01), US , pages 1066 - 1078, XP009548743, ISSN: 2157-6564, DOI: 10.5966/sctm.2014-0079

## Description

### TECHNICAL FIELD

The present invention belongs to the field of medicine, and relates to an application of retinal pigment epithelial cells in relieving or treating corneal endothelial functional decompensation.

### BACKGROUND

The cornea is a transparent tissue located on the anterior of the eyeball, and is divided into five layers, which sequentially comprises epithelial cell layer, bowman membrane, stromal layer, descemet's membrane, and endothelial cell layer from front to back. The highly transparent and optical properties of the cornea are one of the prerequisites that normally exert physiological functions, while corneal endothelial cells play an important role in maintaining the normal physiological function of the cornea. Corneal endothelial cells are single-layer cells in corneal inner layer, which forms a physical barrier between descemet's membrane and aqueous humor, adjusts the concentration and moisture of ions in the cornea by the ion "pump" function to maintain the semi-dehydration state of the cornea, and ensures the normal thickness and transparency of the cornea. Once the function of corneal endothelial cells is disordered, it often leads to corneal edema, causing partial or even complete corneal blindness.

Normal human corneal endothelial cells have extremely limited proliferation capacity in vivo. Endothelial cell damage and loss caused by trauma, inflammation, cataract surgery, etc., can only be filled by the enlargement and migration of surrounding cells. When the density of human corneal endothelial cells drops to its physiological critical value (about 400 -500 cells/mm²), corneal edema occurs and vision loss in severe cases occurs. At present, there are about 4 million patients with corneal blindness in China, including nearly 1 million patients with endothelial blindness. Corneal transplantation is the only clinical therapeutic strategy for corneal endothelial decompensation. Due to the lack of corneal donors in China, only less than 10000 patients regain their sight through corneal transplantation every year, which is far from covering all clinical needs. In order to solve the problem of the corneal donor shortage, the current main corneal endothelial alternative seed cell research comprises cultured adult stem cells such as human corneal endothelial cells and skin progenitor cells, and corneal endothelial cells derived from human embryonic stem cells (hESCs) and human induced pluripotent stem cells (hiPSCs).

Although the transplantation of primary corneal endothelial cells, adult stem cells, or pluripotent stem cells-derived cells can improve corneal endothelial function, the effect is limited. Up to now, there are no ideal corneal endothelial alternative seed cells to achieve long-term corneal transparency and wide clinical application. The team of Japanese scientist Professor Kinoshita transplanted human corneal endothelial cells by anterior chamber injection to clinically treat 11 patients with corneal endothelial dystrophy, and they restored corneal transparency, but the results of 5-year follow-up showed that the corneal endothelium of some patients reappeared pathological "guttae" structure. Cultured human corneal endothelial cells still rely on high-quality donor cornea, and adult corneal endothelial seed cells cannot be expanded in vitro in large quantities, so the source and number of these cells is limited. Adult stem cell-derived alternative cells, such as skin progenitor cells, have poor purity, difficult industrial preparation and limited therapeutic effect; hESC/hiPSC has unlimited proliferative ability and pluripotency, it has been reported that hESC/hiPSC differentiated into neural crest, corneal endothelial precursor and mature corneal endothelioid cells, some experiments have proved that corneal endothelial precursor cells and mature corneal endothelioid cells can be applied to animal models to restore corneal transparency, however, there is currently no standardized method for directed differentiation of hESC/hiPSC to corneal endothelial cells, the long-term efficacy and safety of hESC/hiPSC-derived corneal endothelial cells in vivo remains to be studied. Therefore, to find the ideal corneal endothelial alternative seed cells is still an urgent problem to be solved in the field of corneal endothelial treatment.

### SUMMARY OF THE INVENTION

After a large number of studies, the inventors find that although retinal pigment epithelial cells are greatly different from corneal endothelial cells in terms of tissue differentiation source, anatomical location, and somatic tissue cell function, they have a homologous regular hexagonal morphology and express tight junction proteins, suggesting that retinal pigment epithelial cells have the possibility of providing barrier function as substitutes for corneal endothelial cells and treating corneal endothelial functional decompensation. In order to solve the shortcomings of the prior art, the object of the present invention is to provide corneal endothelial alternative cells; the further objective is to select hESC/hiPSC-derived retinal pigment epithelial cells as seed cells, providing a seed cell source that could be supplied indefinitely and used clinically safely; more further purpose is to improve the function of transplanted cells, optimize the preparation process of cell suspension, and ensure the normal function of transplanted cells.

The present invention provides:
1. Retinal pigment epithelial cells for use in a method of medical treatment as substitutes for corneal endothelial cells.
2. Retinal pigment epithelial cells for use in relieving or treating corneal endothelial injury, corneal endothelial lesion, corneal endothelial cell dysfunction, or corneal endothelial functional decompensation.
3. Retinal pigment epithelial cells for use in an individual suffering from corneal endothelial function decompensation to relieve or treat corneal thickness abnormality, corneal transparency decline, corneal edema, vision decline or loss, eye dryness, or eye pain.

The retinal epithelial cells may be in the form of a retinal pigment epithelial cell suspension, the cell suspension comprising retinal pigment epithelial cells and a DMEM low-sugar culture medium, wherein the ratio of the retinal pigment epithelial cells to the DMEM low-sugar culture medium is 3 × 10⁵ -1.2 × 10⁶: 200 -300 µL; preferably, the ratio of the retinal pigment epithelial cells to the DMEM low-sugar culture medium is 5 × 10⁵ -1 × 10⁶: 200 -300 µL.

In a preferred embodiment of the present invention, the retinal pigment epithelial cells are obtained by differentiation of human embryonic stem cells or human-induced pluripotent stem cells.

In a preferred embodiment of the present invention, the pigment-producing gene Tyrosinase of the human embryonic stem cells or human induced pluripotent stem cells is knocked out.

In a preferred embodiment of the present invention, the cell suspension further comprises one or more specific inhibitors, and the specific inhibitor comprises Y27632, nicotinamide and/or TGF-β inhibitor SB431542.

A method for preparing retinal pigment epithelial cell suspension is disclosed but not explicitly claimed as such, comprising the following steps:
Step 1. directed differentiation: obtaining hESC/hiPSC-derived retinal pigment epithelial cells by using differentiation medium;
Step 2. enzymatic dissociation: treating the hESC/hiPSC-derived retinal pigment epithelial cells in step 1 with cell digestive enzyme, and using complete culture medium to terminate the enzyme reaction;
Step 3. single cell collection: using a pipettor to gently blow cells in step 2 into single cells, collecting the cells into a centrifuge tube, centrifuging and discarding supernatant to retain the cell precipitate;
Step 4. cell suspension preparation: resuspending the cells in step 3 by using DMEM basal culture medium, and dissolving 3 × 10⁵ -1.2 × 10⁶ cells per 200 -300 µL of DMEM basal culture medium to obtain cell suspension.

Preferably, the differentiation medium in step 1 comprises 1: 1 proportion of DMEM/F12 and Neuralbasal Medium, 1- 4mM glutamine, 0.1-1.3mM non-essential amino acid, 0.1 -1.3mM β-mercaptoethanol, and 1% N2 additive;

Preferably, the differentiation culture medium in step 1 comprises DMEM/F12 culture medium, 5 -15% serum substitute, 1-4 mM glutamine, 0.1 -1.3 mM non-essential amino acid, and 0.1 -1.3 mM β-mercaptoethanol.

More preferably, the differentiation medium in step 1 comprises differentiation medium 1 and differentiation medium 2, wherein the differentiation medium 1 comprises DMEM/F12 and Neuralbasal Medium (1: 1), 2 mM glutamine, 0.1 mM non-essential amino acids, 0.1 mM β-mercaptoethanol, and 1% N2 additives; the differentiation medium 2 comprises DMEM/F12 medium, 10% serum substitute, 2 mM glutamine, 0.1 mM non-essential amino acid, and 0.1 mM β-mercaptoethanol. The method of induction differentiation comprises using cell differentiation medium 1 (DMEM/F12 and Neuralbasal medium (1:1), 2 mM glutamine, 0.1 mM non-essential amino acids, 0.1 mM β-mercaptoethanol and 1% N2 supplement) mixed with 2% Matrigel to culture for 2 days, then changing to Matrigel-free medium for 5 days; using differentiation medium 2 (DMEM/F12 medium, 10% serum substitute, 2 mM glutamine, 0.1 mM non-essential amino acid, 0.1 mM β-mercaptoethanol) to culture for 3 weeks; mechanical separation of retinal pigment epithelial cells and cell expansion.

More preferably, the cell digestive enzyme in step 2 is Accutase, and the processing temperature is 37°C; and 5 -15 µm Y27632 is added in step 4.

The use of retinal pigment epithelial cells as substitutes for corneal endothelial cells, refers to use for replacing damaged, diseased or missing corneal endothelial cells.

The retinal pigment epithelial cells provided by the present invention can be administered in any convenient dosage form, and the preferred dosage form comprises injection, cell sheet or kit. Regardless of the dosage form, retinal pigment epithelial cells are administered to the anterior chamber of the individual's eyeballs.

The following 1 to 13 items are disclosed but not explicitly claimed as such:
1. Applications of Retinal Pigment Epithelial Cells as Corneal Endothelial substitute.
2. An application of retinal pigment epithelial cells in the preparation of pharmaceutical composition for relieving or treating corneal endothelial injury, corneal endothelial lesion, corneal endothelial cell dysfunction, and corneal endothelial functional decompensation.
3. An application of retinal pigment epithelial cells in the preparation of pharmaceutical composition for relieving or treating corneal thickness abnormality, corneal transparency decline, corneal edema, vision decline or loss, eye dryness, eye pain of an individual suffering from corneal endothelial functional decompensation.
4. The application of items 1-3, wherein said retinal pigment epithelial cells are administered to the anterior chamber of an individual's eyeballs, and said pharmaceutical composition comprises retinal pigment epithelial cells and DMEM low-sugar culture medium, wherein the ratio of said retinal pigment epithelial cells and DMEM low-sugar culture medium is 3 × 10⁵ -1.2 × 10⁶: 200 -300 micro liters.
5. The application of items 1-4, wherein the ratio of said retinal pigment epithelial cells and DMEM low-sugar culture medium is 5 × 10⁵ -1× 10⁶: 200 -300 micro liters.
6. The application of item 4, said pharmaceutical composition further comprises one or more specific inhibitors, and said specific inhibitors comprise Y27632, nicotinamide and/or TGF-β inhibitor SB431542.
7. The application of items 1-6, said retinal pigment epithelial cells are obtained by differentiation of human embryonic stem cells or human-induced pluripotent stem cells.
8. The application of item 7, the pigment-producing gene Tyrosinase of said human embryonic stem cells or human induced pluripotent stem cells are knocked out.
9. The application of items 1-3, the dosage form of said pharmaceutical composition comprises injection, cell sheet or kit.
10. A method for preparing retinal pigment epithelial cell suspension, comprising the following steps:
   Step 1. directed differentiation: obtaining hESC/hiPSC-derived retinal pigment epithelial cells by using differentiation medium;
   Step 2. enzymatic dissociation: treating the hESC/hiPSC-derived retinal pigment epithelial cells in step 1 with cell digestive enzyme, and using complete culture medium to terminate the enzyme reaction;
   Step 3. single cell collection: using a pipettor to gently blow cells in step 2 into single cells, collecting the cells into a centrifuge tube, centrifuging and discarding supernatant to retain the cell precipitate;
   Step 4. cell suspension preparation: resuspending the cells in step 3 by using DMEM basal culture medium, and dissolving every 3 × 10⁵ -1.2 × 10⁶ cells in 200 -300 µL of DMEM basal culture medium to obtain cell suspension.
11. The method of item 10, said differentiation medium in step 1 comprises differentiation medium 1 and differentiation medium 2, wherein the differentiation medium 1 comprises DMEM/F12, Neuralbasal Medium, glutamine, non-essential amino acids, β-mercaptoethanol, and N2 additives; said differentiation medium 2 comprises DMEM/F12 medium, serum substitute, glutamine, non-essential amino acid, and β-mercaptoethanol; said differentiation culture medium 1 is mixed with the Neuralbasal Medium culture medium in a 1: 1 proportion.
12. The method of items 10-11, step 1 comprises using cell differentiation medium 1 (DMEM/F12 and Neuralbasal medium (1:1), 2 mM glutamine, 0.1 mM non-essential amino acids, 0.1 mM β-mercaptoethanol and 1% N2 supplement) mixed with 2% Matrigel to culture for 2 days, then changing to Matrigel-free medium for 5 days; using differentiation medium 2 (DMEM/F12 medium, 10% serum substitute, 2 mM glutamine, 0.1 mM non-essential amino acid, 0.1 mM β-mercaptoethanol) to culture for 3 weeks; mechanical separation of retinal pigment epithelial cells and cell expansion.
13. The method of items 10-12, said cell digestive enzyme in step 2 is Accutase, and the processing temperature is 37°C; and said cell suspension in step 4 also comprises 5 -15 µm Y27632.

The beneficial technical effects of the present invention are as follows:
According to the invention, retinal pigment epithelial cells are provided for use as alternative seed cells of corneal endothelium for the first time; the cell suspension and the preparation method can effectively replace corneal endothelial function to recover corneal transparency and corneal thickness while ensuring cell viability. In addition, the seed cells for replacing corneal endothelium provided by the present invention can be obtained by inducing differentiation from hESC/hiPSCs, and are infinitely supplied, and the application security thereof has been reported in existing clinical experiments. According to the preparation method disclosed herein and the transplantation method using the retinal pigment epithelial cells for use of the invention, highly specialized equipment, reagents or skills are not needed, so that researchers and clinical personnel can operate conveniently, and therefore, the invention has wide application values and positive social benefits.

### BRIEF DESCRIPTION OF THE DRAWINGS

Figure 1 shows the cell morphology diagram of the hESC-derived retinal pigment epithelial cells and the staining diagram of symbolic gene in Example 1.
Figure 2 shows the general image and OCT photos of the cornea at 1st day, 3rd day, 7th day and 14th day after retinal pigment epithelial cell transplantation in Example 1, showing corneal transparency and thickness.
Figure 3 shows the general image and OCT photos of the cornea at 1st day, 3rd day, 7th day and 14th day after retinal pigment epithelial cell transplantation in Example 2, showing corneal transparency and thickness.

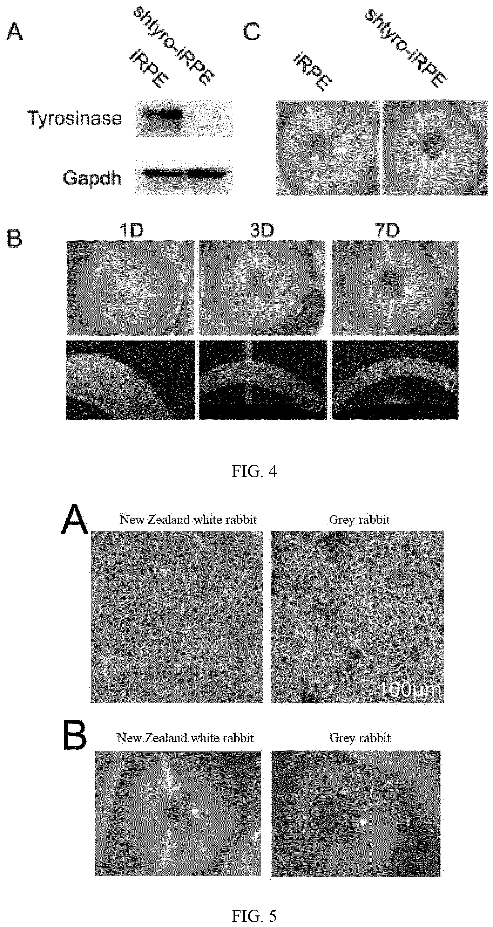

Figure 4 shows the identification of the knockout of the pigment-producing gene Tyrosinase in retinal pigment epithelial cells and corneal endothelial repair function in vivo in Example 3. (A) gene expression of retinal pigment epithelial cell before and after Tyrosinase knockout; iRPE: normal induced retinal pigment epithelial cells, shtyro-iRPE: induced retinal pigment epithelial cells with Tyrosinase knockout; (B) general image and OCT photographs of the cornea at 1st day, 3rd day, and 7th day after shtyro-iRPE transplantation; (C) general image of the corneal 1 month after transplantation of normal cells and knockout cells.
Figure 5 shows the repair effect of rabbit primary retinal pigment epithelial cells on corneal endothelial functional decompensation in Example 4. (A) photos of isolated and cultured retinal pigment epithelial cells from New Zealand white rabbits and grey rabbits; (B) general image of the cornea from New Zealand white rabbits and grey rabbits at 7th day after retinal pigment epithelial cell transplantation.
Figure 6 general image of the cornea at 7th day after transplantation in Comparative Example 2.
Figure 7 general image of the cornea at 7th day after transplantation in Comparative Example 3.

### DETAILED DESCRIPTION OF THE EMBODIMENTS

The present invention is further illustrated by the following embodiments explaining the present invention, the following embodiments are only used to illustrate the present invention and should not be regarded as limiting the scope of the present invention. Unless otherwise indicated, the technical and scientific terms used herein are generally understood by those of ordinary skill in the art to which the invention belongs. If the specific conditions are not indicated in the embodiment, the conditions recommended by the manufacturer shall be carried out in accordance with the general conditions or the conditions recommended by the manufacturer. The reagents or instruments used are conventional products that are commercially available if the manufacturer is not identified.

In the embodiments of the present invention, the hESC cell line H1 was donated by Professor Yin Zhengqin's laboratory; The hiPSC cell line DY0100 was purchased from the Chinese Academy of Sciences Cell Bank/Stem Cell Bank; Tryosinase-specific knockout hESC H1 cell line: Tryosinase-specific knockout virus were purchased from Shanghai GK Gene Medical Technology Co., Ltd. and the Tryosinase-specific knockout hESC H1 cell line was prepared according to its instruction; New Zealand white rabbits and grey rabbits were purchased from Xilingjiao Breeding Center in Jinan.

### Example 1 (hESC cell line H1)

### (1) Directed differentiation

Based on the differentiation method priorly disclosed (Rapid Differentiation of Multi-Zone Ocular Cells from Human Induced Pluripotent Stem Cells and Generation of Corneal Epithelial and Endothelial Cells, Stem Cells Dev. 2019 Apr 1; 28(7): 454-463), hESC cell line H1 cells were cultured till fusion rate reached approximately 80% using mTeSR1 medium, then digested with 5 mg/ml type IV collagenase for 15 mins and seeded into 1% Matrigel-coated dish; cell differentiation medium 1 (DMEM/F12 and Neuralbasal medium (1:1), 2 mM glutamine, 0.1 mM non-essential amino acids, 0.1 mM β-mercaptoethanol and 1% N2 supplement) mixed with 2% Matrigel was utilized to culture for 2 days, then changed to Matrigel-free medium for 5 days; using differentiation medium 2 (DMEM/F12 medium, 10% serum substitute, 2 mM glutamine, 0.1 mM non-essential amino acid, 0.1 mM β-mercaptoethanol) to culture for 3 weeks; mechanical separation of retinal pigment epithelial cells and cell expansion.

### (2) Enzymatic digestion of cells

hESC-derived retinal pigment epithelial cells were treated at 37°C by Accutase enzyme for 10-20 mins, and the enzyme reaction was terminated by complete medium; cells were gently pipetted into single cells and collected into 15 ml centrifuge tubes, then centrifuged at 1000 rpm for 3 mins; the supernatant was discarded and the precipitate was retained.

### (3) Preparation of cell suspension

Cells were resuspended using DMEM low-glucose basal medium, the number of cells were counted by cell counter for distribution. Every 5×10⁵∼1×10⁶ cells were dissolved in 200-300 µl DMEM basal medium in addition of 10 µM Y27632 for transplantation.

### (4) Injection of cell suspension into the anterior chamber

Ketamine hydrochloride (40 mg/kg) and chlorpromazine hydrochloride (20 mg/kg) was intramuscularly administrated to anesthetize 10 New Zealand white rabbits. The right eye was washed after the eyelids were opened by eye speculum. A lateral incision of about 2mm was made at 10 o'clock spot at the corneal limbus, and carbacholine was injected into the anterior chamber to shrink the pupil. Sodium hyaluronate was injected from the lateral incision to stabilize the anterior chamber. Autologous corneal endothelial cells within a diameter of 7~9 mm in the center of the eye were scraped with a 20-gauge silicone needle. The scraped cell fragments and the residual sodium hyaluronate in the anterior chamber were washed with normal saline. 1:10 heparin sodium injection was injected to prevent anterior chamber exudation, and 10-0 nylon thread was intermittently sutured to the limbal lateral incision.

The cell suspension was injected into the anterior chamber of the right eye from the limbus into the anterior chamber using a 1 ml syringe and tobramycin dexamethasone eye ointment was applied to cover the eye. The rabbit was held in the side-lying position under anesthesia for 3 hours to keep the right eye downward in order to facilitate rapid attachment of transplanted cells. 10 mM Y-27632 was given four times daily postoperatively. After one week change to 1 mM Y-27632 four times daily, while tobramycin dexamethasone eye drops four times daily and cyclosporine eye drops twice daily were given.

### (5) Functional evaluation

After surgery, the recovery of corneal transparency was observed by slit lamp microscopy, the morphology and density of transplanted corneal endothelial cells were evaluated by living confocal corneal microscopy, and the change of corneal thickness was measured by ultrasound corneal thickness gauge.

### Results & Analysis:

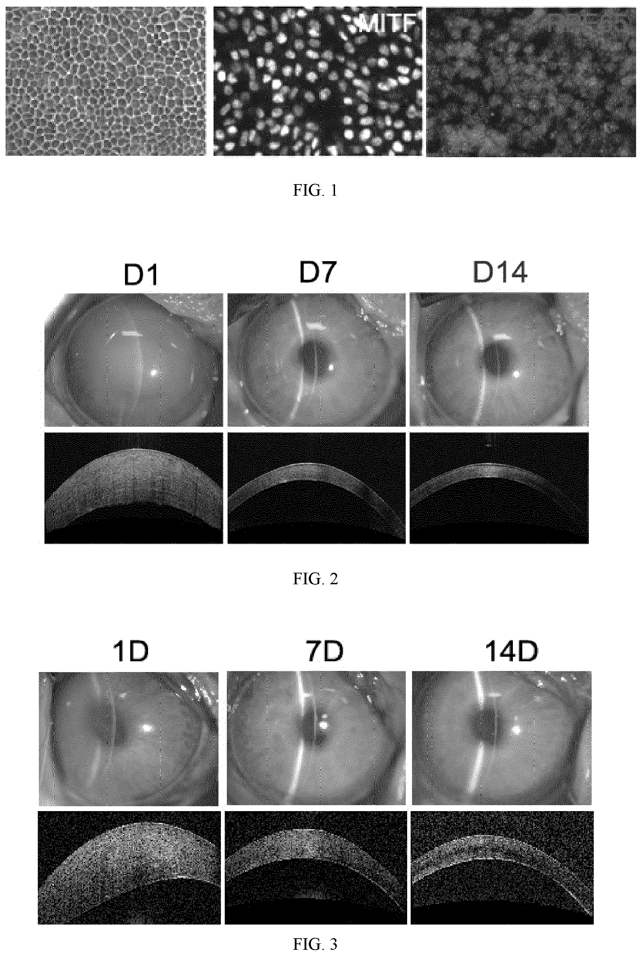

Based on the present example, the differentiation from the hESC cell line H1 into retinal pigment epithelial cells can be induced (Figure 1); Transplantation of hESC-derived retinal pigment epithelial cells restored corneal clarity and corneal thickness within 7 days and remained corneal transparency till 14 days (Figure 2). The results suggest that hESC-derived retinal pigment epithelial cells can replace the function of corneal endothelial cells and quickly restore corneal transparency.

### Example 2 (hiPSC cell line DY0100)

This example used the hiPSC cell line DY0100 to induce retinal pigment epithelial cells.

### (1) Directed differentiation

The induction differentiation method was the same as Example 1, using mTeSR1 medium to culture hiPSC cell line DY0100 till cell fusion reach approximately 80%, then using 5mg/ml type IV collagenase to digest for 15 minutes, inoculating into 1% Matrigel-coated dishes, and using cell differentiation medium 1 mixed with 2% Matrigel to culture for 2 days, changing to Matrigel-free medium for 5 days; using differentiation medium 2 to culture for 3 weeks; mechanical separation of retinal pigment epithelial cells and cell expansion.

### (3) Preparation of cell suspension

Cells were resuspended using DMEM low-glucose basal medium, the number of cells were counted by cell counter for distribution. Every 8×10⁵∼1×10⁶ cells were dissolved in 200-300 µl DMEM basal medium with addition of 10 µM Y27632 and SmM nicotinamide for transplantation.

(2) Enzymatic digestion of cells, (4) Injection of cell suspension into the anterior chamber and (5) Functional evaluation were the same as Example 1.

### Results & Analysis:

Based on the present example, the differentiation of the hiPSC cell line DY0100 into retinal pigment epithelial cells can be induced; Transplantation of hiPSC-derived retinal pigment epithelial cells restored corneal clarity and corneal thickness within 7 days and remained corneal transparent till 14 days (Figure 3).

### Example 3 (knockout of the chromogenic gene Tyrosinase)

In order to reduce pigmentation, the present example knocked out the chromogenic gene Tyrosinase, and prepared unpigmented hESC/hiPSC-RPE cells, which can also maintain corneal transparency after transplantation. The present embodiment used CRISPR-Cas9 technology to specifically knock out the Tryosinase gene to prepare pigment-free retinal pigment epithelial cells.

In some embodiments, the hES cell line H1 was used; In some other embodiments, the hiPS cell line DY0100 was used.

### (1) Construction of Tryosinase knockout cells:

After digestion of the hES cell line H1 or hiPS cell line DY0100 which reached about 80% cell fusion, the cells were inoculated according to the ratio of 1:20~30, and transfection reagent mixed with siRNA was added in the next day, transfection was performed when the confluence reached about 50-60% after 16-24 hours culture, and the amount of the added virus = (MOI× number of cells) / virus titers. After 12-20 hours of transfection, change to mTeSR1 complete medium for 72-96 hours culture, and the transfection performance was evaluated according to fluorescence intensity. The sets with most fluorescent signals were selected for flow cytometry sorting, culture, and expansion to establish Tryosinase knockout cell lines.

(2) Induction of differentiation, (3) Enzymatic digestion of cells, (4) Preparation of cell suspension, (5) Injection of cell suspension into the anterior chamber and (6) Functional evaluation were the same as Example 1.

### Results & Analysis:

Knockout of the chromogenesis-related gene Tyrosinase in hESC/hiPSC-derived retinal pigment epithelial cells was achieved by the method described in the present example (Figure 4A); After transplantation, genetically modified retinal pigment epithelial cells can quickly restore corneal transparency and reduce corneal thickness (Figure 4B); Corneal transparency and corneal thickness were maintained one month after transplantation with no pigmentation (Figure 4C).

### Example 4 (isolation and culture of primary retinal pigment epithelial cells derived from New Zealand white rabbits and gray rabbits)

In some embodiments, primary unpigmented retinal pigment epithelial cells of New Zealand white rabbit cultured in vitro were used; In some other embodiments, pigmented retinal pigment epithelial cells of gray rabbit cultured in vitro were used.

### (1) Isolation and culture

2-4 weeks old New Zealand white rabbits and gray rabbits were used, sacrificed by air embolization method; eyeballs were removed under sterile conditions, soaked in 1000u gentamicin saline at 4°C for 30 minutes, and then replaced in normal saline for 3 hours. Cut the anterior segment and neuroretinal epithelium under a dissecting microscope, put the posterior eye cup into a 12-well plate Petri dish, added 0.25% pancreatic enzyme to about 3/4 eye cup, put it into a 37 °C incubator for 30 min for digestion, added a stop solution to terminate digestion, gently pipetted to detach RPE cells, then collected, centrifuged and inoculated; DMEM/F12 medium containing 10% fetal bovine serum was used to culture, and the solution was refreshed every two days for approximately 2 weeks, and identification was performed by morphology, PCR, and staining.

### (3) Preparation of cell suspension

Cells were resuspended using DMEM low-glucose basal medium, the number of cells was counted by cell counter for distribution. Every 5×10⁵~8×10⁶ cells were dissolved in 200-300 µl DMEM basal medium in addition of 10 µM Y27632 for transplantation.

(2) Enzymatic digestion of cells, (4) Injection of cell suspension into the anterior chamber and (5) Functional evaluation were the same as Example 1.

### Results & Analysis:

The preparation of primary retinal pigment epithelial cells of New Zealand white rabbits and gray rabbits was achieved by the method described in the present example, which exhibited a regular cell morphology (Figure 5A); Corneal transparency can be quickly restored 7 days after cell transplantation (Figure 5B).

### Comparative Example 1 (Pancreatin/Collagenase)

### (1) Induction of differentiation: steps were the same as Example 1.

### (2) Enzymatic digestion of cells

hESC-derived retinal pigment epithelial cells were treated at 37°C by 0.25% pancreatin for 3~10 mins, or hESC-derived retinal pigment epithelial cells were treated at 37°C by 5mg/ml IV collagenase for 5-15 mins, and the enzyme reaction was terminated by complete medium; cells were gently pipetted into single cells and collected into 15 ml centrifuge tubes, then centrifuged at 1000 rpm for 3 mins; the supernatant was discarded and the precipitate was retained.

### (3) Cell viability and size detection

Trypan blue staining was performed and cell viability, size, etc. were counted by cell counter.

### Results & Analysis:

The mortality rate of cells obtained by pancreatic digestion was high, and that by collagenase digestion was not easy to dissociate to obtain single cells, and the digestion time was too long, so pancreatase and collagenase can be used for enzymatic digestion of cells, but Accutase is preferred.

### Comparative Example 2 (DMEM High Sugar Medium)

In this example, cell suspension was prepared using DMEM high sugar medium (containing 4.5 g/ml glucose).
(1) Induction of differentiation, (2) Enzymatic digestion of cells, (4) Injection of cell suspension into the anterior chamber and (5) Functional evaluation were the same as Example 1.

### (3) Preparation of cell suspension

Cells were resuspended using DMEM high sugar medium (containing 4.5 g/ml glucose), the number of cells were counted by cell counter for distribution. Every 5×10⁵∼1×10⁶ cells were dissolved in 200-300 µl DMEM high sugar medium for transplantation.

### Results & Analysis:

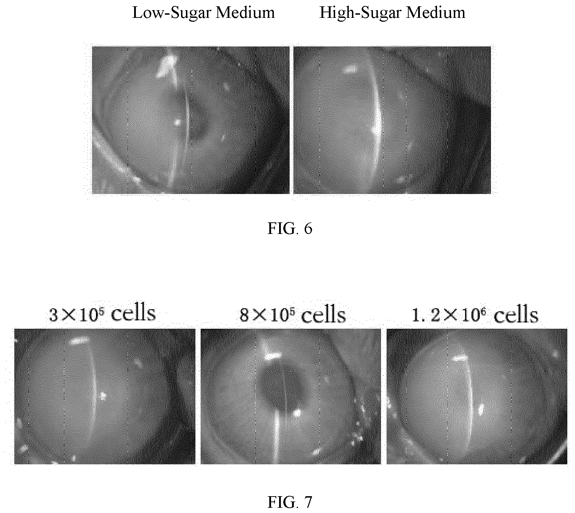

After transplantation of cell suspension resuspended in DMEM high-glucose medium as a solvent, the anterior chamber exudation was severe, and cornea edema lasted, and corneal transparency did not restore (Figure 6).

### Comparative Example 3

The present example prepares cell suspensions with different cell volumes.
(1) Induction of differentiation, (2) Enzymatic digestion of cells, (4) Injection of cell suspension into the anterior chamber and (5) Functional evaluation were the same as Example 1.
(3) Preparation of cell suspensions

Resuspend cells using DMEM low-glucose basal medium, count the number of cells by cell counter for distribution. Dissolve every 1.5×10⁶∼1×10⁶, 1×10⁶∼5×10⁵, 5×10⁵∼1×10⁵ cells in 200-300 µl DMEM basal medium respectively for transplantation.

### Results & Analysis:

Postoperative evaluation found that severe exudation of the anterior chamber and persistent cornea edema occurred after transplantation with over 1.2×10⁶ cells; while persistent cornea edema occurred and cornea transparency recovery failed after transplantation with less than 3×10⁵ cells (Figure 7).

## Claims

1. Retinal pigment epithelial cells for use in a method of medical treatment as substitutes for corneal endothelial cells.

2. Retinal pigment epithelial cells for use in relieving or treating corneal endothelial injury, corneal endothelial lesion, corneal endothelial cell dysfunction, or corneal endothelial functional decompensation.

3. Retinal pigment epithelial cells for use in an individual suffering from corneal endothelial function decompensation to relieve or treat corneal thickness abnormality, corneal transparency decline, corneal edema, vision decline or loss, eye dryness, or eye pain.

4. Retinal pigment epithelial cells for use according to any one of claims 1-3, wherein in said use said retinal pigment epithelial cells are administered to the anterior chamber of an individual's eyeballs in the form of a pharmaceutical composition , which comprises retinal pigment epithelial cells and DMEM low-sugar culture medium, wherein the ratio of said retinal pigment epithelial cells and DMEM low-sugar culture medium is 3 × 10⁵ -1.2 × 10⁶: 200 -300 micro liters.

5. Retinal pigment epithelial cells for use according to claim 4, wherein the ratio of said retinal pigment epithelial cells and DMEM low-sugar culture medium is 5 × 10⁵ - 1 × 10⁶: 200 -300 micro liters.

6. Retinal pigment epithelial cells for use according to claim 4, wherein said pharmaceutical composition also comprises one or more specific inhibitors, and said specific inhibitor comprises Y27632, nicotinamide and/or TGF-β inhibitor SB431542.

7. Retinal pigment epithelial cells for use according to any one of claims 1-6 wherein said retinal pigment epithelial cells are obtained by differentiation of human embryonic stem cells or human induced pluripotent stem cells.

8. Retinal pigment epithelial cells for use according to claim 7, wherein the pigment-producing gene Tyrosinase of said human embryonic stem cells or human induced pluripotent stem cells is knocked out.

9. Retinal pigment epithelial cells for use according to any one of claims 1-3, wherein in said use said retinal pigment epithelial cells are in the form of a cell suspension, cell sheet or kit.

## Patentansprüche

1. Netzhautpigmentepithelzellen zur Verwendung in einem Verfahren zur medizinischen Behandlung als Ersatz für Hornhautendothelzellen.

2. Netzhautpigmentepithelzellen zur Verwendung bei der Linderung oder Behandlung einer Hornhautendothelverletzung, Hornhautendothelläsion, Hornhautendothelzellendysfunktion oder funktionellen Hornhautendotheldekompensation.

3. Netzhautpigmentepithelzellen zur Verwendung in einem Individuum, das an funktioneller Hornhautendotheldekompensation leidet, um eine Hornhautdickeanomalie, Hornhauttransparenzverringerung, Hornhautödem, schlechter werdendes Sehvermögen oder Sehverlust, Augentrockenheit oder Augenschmerzen zu lindern oder zu behandeln.

4. Netzhautpigmentepithelzellen zur Verwendung nach einem der Ansprüche 1 bis 3, wobei in der Verwendung die Netzhautpigmentepithelzellen an die vordere Kammer der Augäpfel eines Individuums in Form einer pharmazeutischen Zusammensetzung verabreicht werden, die Netzhautpigmentepithelzellen und zuckerarmes DMEM-Kulturmedium umfasst, wobei das Verhältnis der Netzhautpigmentepithelzellen zu dem zuckerarmen DMEM-Kulturmedium 3 × 10⁵ - 1,2 × 10⁶:200 - 300 µl beträgt.

5. Netzhautpigmentepithelzellen zur Verwendung nach Anspruch 4, wobei das Verhältnis der Netzhautpigmentepithelzellen zu dem zuckerarmen DMEM-Kulturmedium 5 × 10⁵ - 1 × 10⁶:200 - 300 µl beträgt.

6. Netzhautpigmentepithelzellen zur Verwendung nach Anspruch 4, wobei die pharmazeutische Zusammensetzung auch einen oder mehrere spezifische Inhibitoren umfasst und der spezifische Inhibitor Y27632, Nicotinamid und/oder TGF-β-Inhibitor SB431542 umfasst.

7. Netzhautpigmentepithelzellen zur Verwendung nach einem der Ansprüche 1 bis 6, wobei die Netzhautpigmentepithelzellen durch Differenzierung von menschlichen embryonischen Stammzellen oder menschlichen induzierten pluripotenten Stammzellen erhalten werden.

8. Netzhautpigmentepithelzellen zur Verwendung nach Anspruch 7, wobei das pigmentproduzierende Gen Tyrosinase der menschlichen embryonischen Stammzellen oder der menschlichen induzierten pluripotenten Stammzellen ausgeknockt ist.

9. Netzhautpigmentepithelzellen zur Verwendung nach einem der Ansprüche 1 bis 3, wobei in der Verwendung die Netzhautpigmentepithelzellen in Form einer Zellsuspension, Zellschicht oder als Set vorliegen.

## Revendications

1. Cellules épithéliales pigmentaires rétiniennes à utiliser dans un procédé de traitement médical en tant que substituts de cellules endothéliales cornéennes.

2. Cellules épithéliales pigmentaires rétiniennes à utiliser pour soulager ou traiter un traumatisme de l'endothélium cornéen, une lésion endothéliale cornéenne, un dysfonctionnement des cellules endothéliales cornéennes ou une décompensation fonctionnelle de l'endothélium cornéen.

3. Cellules épithéliales pigmentaires rétiniennes à utiliser chez un individu souffrant de décompensation de la fonction endothéliale cornéenne pour soulager ou traiter une anomalie de l'épaisseur de la cornée, un déclin de la transparence de la cornée, un œdème cornéen, un déclin ou une perte de vision, une sécheresse oculaire ou une douleur oculaire.

4. Cellules épithéliales pigmentaires rétiniennes à utiliser selon l'une quelconque des revendications 1 à 3, dans lesquelles, dans ladite utilisation, lesdites cellules épithéliales pigmentaires rétiniennes sont administrées à la chambre antérieure des globes oculaires d'un individu sous la forme d'une composition pharmaceutique, qui comprend des cellules épithéliales pigmentaires rétiniennes et un milieu de culture à faible teneur en sucre DMEM, dans lequel le rapport desdites cellules épithéliales pigmentaires rétiniennes et du milieu de culture à faible teneur en sucre DMEM est de 3 × 10⁵ -1,2 × 10⁶ : 200-300 microlitres.

5. Cellules épithéliales pigmentaires rétiniennes à utiliser selon la revendication 4, dans lesquelles le rapport desdites cellules épithéliales pigmentaires rétiniennes et du milieu de culture à faible teneur en sucre DMEM est de 5 × 10⁵ -1 × 10⁶ : 200-300 microlitres.

6. Cellules épithéliales pigmentaires rétiniennes à utiliser selon la revendication 4, dans lesquelles ladite composition pharmaceutique comprend également un ou plusieurs inhibiteurs spécifiques, et ledit inhibiteur spécifique comprend Y27632, nicotinamide et/ou inhibiteur de TGF-β SB431542.

7. Cellules épithéliales pigmentaires rétiniennes à utiliser selon l'une quelconque des revendications 1 à 6, dans lesquelles lesdites cellules épithéliales pigmentaires rétiniennes sont obtenues par différenciation de cellules souches embryonnaires humaines ou de cellules souches pluripotentes induites par l'Homme.

8. Cellules épithéliales pigmentaires rétiniennes à utiliser selon la revendication 7, dans lesquelles le gène Tyrosinase producteur de pigment desdites cellules souches embryonnaires humaines ou cellules souches pluripotentes induites par l'Homme est éliminé.

9. Cellules épithéliales pigmentaires rétiniennes à utiliser selon l'une quelconque des revendications 1 à 3, dans lesquelles, dans ladite utilisation, lesdites cellules épithéliales pigmentaires rétiniennes sont sous la forme d'une suspension cellulaire, d'une feuille cellulaire ou d'un kit.
